# EUROPEAN PATENT APPLICATION

(11) **EP 2 085 027 A2**
(43) Date of publication of application: **05.08.2009**
(21) Application number: 09001114.9
(22) Date of filing: 27.01.2009
(51) Int. Cl.: A61B 5/044

(54) **Biological information display method and biological information display apparatus**

(30) Priority: 30.01.2008 JP 2008019593
(71) Applicant: Nihon Kohden Corporation, Shinjuku-ku Tokyo (JP)
(72) Inventor: Kamataki, Osamu c/o Nihon Kohden Corporation, Tokyo (JP)
(74) Representative: Grünecker, Kinkeldey, Stockmair & Schwanhäusser Anwaltssozietät

(57) **Abstract**

A method of displaying a plurality of ST values constituting biological information of a subject displayed on a screen, includes: defining color gradations corresponding to degrees of the plurality of ST values to be acquired; acquiring the plurality of ST values from the subject; determining display elements for the acquired plurality of ST values in accordance with the defined color gradations; and displaying the determined display elements along a time axis in a belt-like form on the screen, for each of the plurality of ST values.

## Description

### BACKGROUND OF THE INVENTION

The present invention relates to a method and apparatus for displaying 12-lead ST measurement values constituting electrocardiogram information which is biological information on one screen.

As a display example of 12-lead ST measurement values of electrocardiogram information, there is the following display (see WO/2004/023994).

WO/2004/023994 discloses the followings.

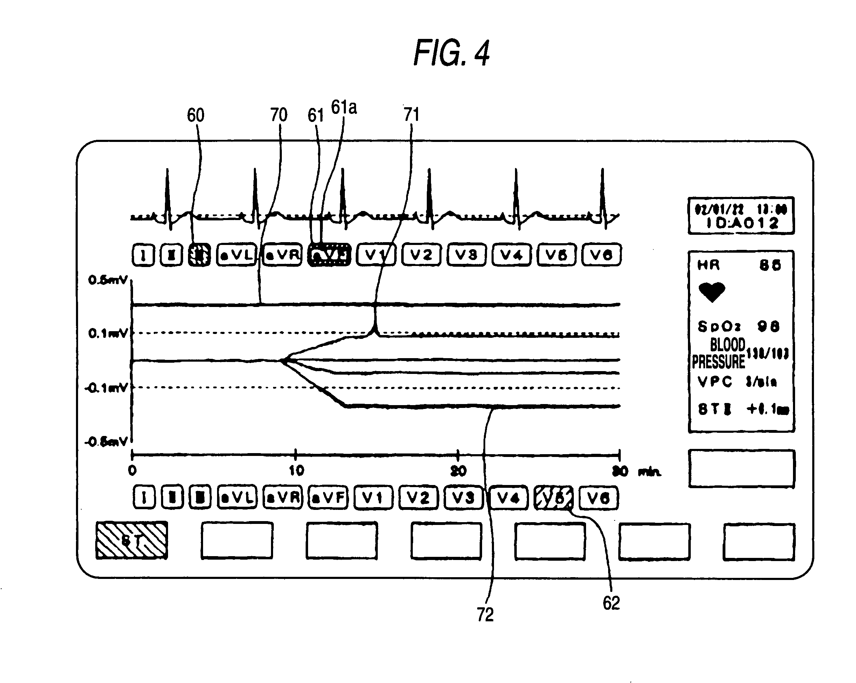

For example, Fig. 4 shows the case where several lead waveform-derived ST levels in 12 leads have an abnormal value. Hereinafter, the abnormal values will be described.

The line 70 indicating the ST level of the lead waveform of lead III exceeds the upper threshold, and is plotted in an abnormal value color X (graph display mode).

In the figure, in order to indicate that the lead III-derived ST level exceeds the upper threshold, a lead icon 60 (for lead III) in an upper portion of the display is displayed in the abnormal value color X in the same manner as the line 70 (the mode of displaying the kind of data is made identical with the graph display mode of the data).

The line 72 indicating the ST level of the lead waveform of lead V5 exceeds the lower threshold, and the portion of the line which exceeds the lower threshold is plotted in an abnormal value color Y.

In the figure, in order to indicate that the lead V5-derived ST level exceeds the lower threshold, a lead icon 62 (for lead V5) in a lower portion of the display is displayed in the abnormal value color Y in the same manner as the line 72.

The line 71 indicating the ST level of the lead waveform of lead aVF has a portion which exceeds the upper threshold, and the portion of the line which exceeds the upper threshold is plotted in an abnormal value color Z.

In the figure, in order to indicate that the lead aVF-derived ST level exceeds the upper threshold, an outer frame 61 of a lead icon (for lead aVF) in an upper portion of the display is displayed in the abnormal value color Z in the same manner as the portion of the line 71 which exceeds the upper threshold.

However, the current lead aVF-derived ST level returns to the normal value range, and therefore an inner frame 61a of the lead icon is displayed in a normal value color.

In the technique disclosed in WO/2004/023994, as shown in Fig. 4, plural ST measurement values are displayed along a time axis in the form of a line graph. Therefore, plural ST measurement values are tangled, and it is not easy to know the variation of each of the plural ST measurement values.

### SUMMARY

It is therefore an object of the invention to provide a method and apparatus for collectively displaying 12-lead ST measurement values in which 12-lead ST measurement values of electrocardiogram information are displayed on a same display screen in a mutually distinguishable manner, so that it is easy to know which lead (portion of the heart) a disease such as ischemic heart disease occurs in.

In order to achieve the object, according to the invention, there is provided a method of displaying a plurality of ST values constituting biological information of a subject displayed on a screen, the method comprising:
defining color gradations corresponding to degrees of the plurality of ST values to be acquired;
acquiring the plurality of ST values from the subject;
determining display elements for the acquired plurality of ST values in accordance with the defined color gradations; and
displaying the determined display elements along a time axis in a belt-like form on the screen, for each of the plurality of ST values.

The biological information may be electrocardiogram information, and the plurality of ST values may be 12-lead ST measurement values.

The plurality of ST values may include a normal ST value and an abnormal ST value, and the color gradation of the abnormal ST value may be clearly different from the color gradation of the normal ST value.

In order to achieve the object, according to the invention, there is also provided a biological information display apparatus which is configured to display 12-lead ST measurement values constituting electrocardiogram information of a subject on a screen, the biological information display apparatus comprising:
a color gradation setting unit, configured to define color gradations corresponding to degrees of the 12-lead ST measurement values to be acquired;
an ST measurement value acquiring unit, configured to acquire the 12-lead ST measurement values from the subject;
a color gradation allocating unit, configured to determine display elements for the acquired 12-lead ST measurement values in accordance with the defined color gradations; and
a displaying unit, configured to display the determined display elements along a time axis in a belt-like form on the screen, for each of the 12-lead ST measurement values.

### BRIEF DESCRIPTION OF THE DRAWINGS

Fig. 1 is a block diagram showing the configuration of the apparatus for collectively displaying 12-lead ST measurement values constituting electrocardiogram information according to the invention.
Fig. 2 is a view showing a state where 12-lead ST measurement values constituting electrocardiogram information according to the invention are collectively displayed on a same screen.
Fig. 3 is a diagram enlargedly showing a part of the ST measurement values of one of the 12 leads (in this case, lead waveform (III)) on the time axis.
Fig. 4 is a view showing a case where several lead waveform-derived ST levels in related-art 12 leads have an abnormal value.

### DETAILED DESCRIPTION OF PREFERRED EMBODIMENTS

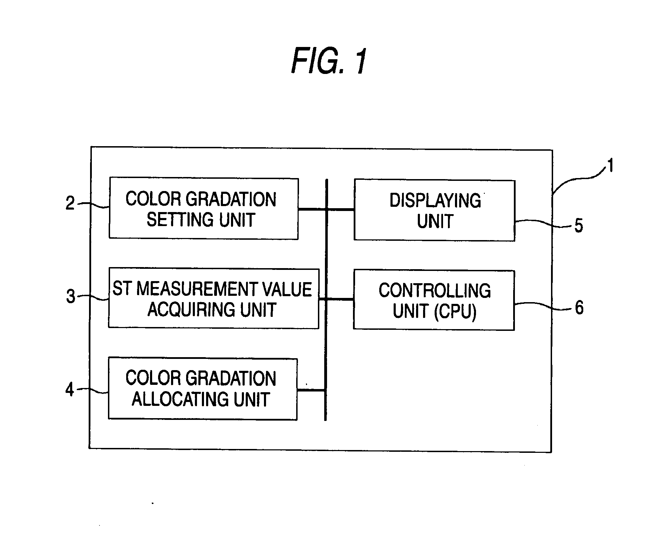

Next, the method and apparatus for collectively displaying 12-lead ST measurement values constituting electrocardiogram information according to the invention will be described with reference to Figs. 1 to 3.

Fig. 1 is a block diagram showing the configuration of the apparatus for collectively displaying 12-lead ST measurement values constituting electrocardiogram information according to the invention.

The reference numeral 1 denotes an electrocardiogram information collective display apparatus which is to be disposed in a biological information acquisition apparatus (not shown) (for example, an electrocardiogram information acquisition apparatus), and which collectively displays 12-lead ST measurement values constituting electrocardiogram information according to the information on a same screen.

The electrocardiogram information collective display apparatus 1 of the invention is configured by: a color gradation setting unit 2 for defining a predetermined color gradation corresponding to the degree of each of 12-lead ST values; an ST measurement value acquiring unit 3 for measuring electrocardiogram potentials of the patient to acquire the potentials as 12-lead ST measurement values; a color gradation allocating unit 4 for determining display elements of the color gradation which is defined by the color gradation setting unit 2, for the acquired 12-lead ST measurement values; a displaying unit 5 for displaying the determined display elements of the color gradation along a time axis in a belt-like form on a display screen, for each of the 12-lead ST measurement values; and a controlling unit (CPU) 6.

The color gradation setting unit 2 defines a predetermined color gradation corresponding to the degree of each of ST values, in the following manner.

In the case where ST values are indicated in the unit of mV, in the range from -2.55 to +2.55 mV, 256 (or more) colors are allocated in accordance with the degree of each ST value, and the values are expressed as a change of the color gradation.

In the case where ST values are indicated in the unit of mm, in the range from -25.5 to +25.5 mm, 256 (or more) colors are allocated in accordance with the degree of each ST value, and the values are expressed as a change of the color gradation.

In the allocation of color gradations to ST values, preferably, a color gradation which is easily distinguishable from others is allocated for an ST value corresponding to an abnormal value.

However, actual ST values often change in a very small portion of the measurement range. In a small range such as that from -0.50 to +0.50 mV, therefore, color gradations may be defined in smaller steps as compared with the other range.

The ST measurement value acquiring unit 3 performs a calculation process on electrocardiogram potentials measured by ECG electrodes which are attached to the body of the patient (for example, six places for chest leads of the patient, and four places for limb leads) to acquire 12-lead ST measurement values constituting electrocardiogram information configured by standard 12 leads , that is, limb 6 lead waveforms (I, II, III, aVR, aVL, and aVF) and chest 6 lead waveforms (V1, V2, V3, V4, V5, and V6).

The color gradation allocating unit 4 allocates color gradations of the display elements which are defined by the color gradation setting unit 2, for the 12-lead ST measurement values which are acquired in the ST measurement value acquiring unit 3.

The displaying unit 5 is a display portion which displays measurement results of the biological information acquisition apparatus (for example, an electrocardiogram information acquisition apparatus). Usually, the displaying unit is configured integrally with the biological information acquisition apparatus, or alternatively may be disposed separately from the biological information acquisition apparatus (for example, in a nurse station).

The controlling unit (CPU) 6 performs a control for collectively displaying electrocardiogram information according to the invention on the displaying unit 5, in accordance with the outputs of the color gradation setting unit 2, the ST measurement value acquiring unit 3, and the color gradation allocating unit 4.

In Fig. 1, the controlling unit (CPU) is shown as a unit inherent in the collective display apparatus 1. Alternatively, the function of the unit may be performed by a controlling unit (CPU) of the biological information acquisition apparatus.

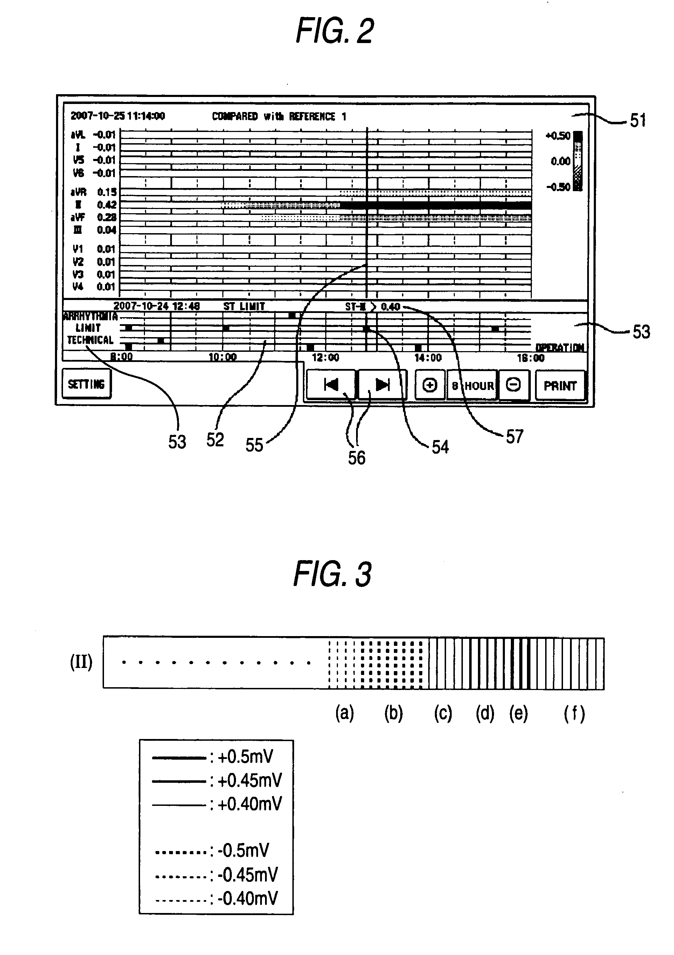

Next, the procedure of collectively displaying 12-lead ST measurement values constituting electrocardiogram information according to the invention, on a same screen will be described with reference to Fig. 2.

Fig. 2 is a view showing a state where 12-lead ST measurement values constituting electrocardiogram information peculiar to the invention and to be displayed on the displaying unit 5 of Fig. 1 are collectively displayed on a same screen.

In Fig. 2, limb 6 lead waveforms (I, II, III, aVR, aVL, and aVF) and chest 6 lead waveforms (V1, V2, V3, V4, V5, and V6) which are 12-lead ST measurement values constituting electrocardiogram information according to the invention are collectively displayed on an ST characteristic display portion 51.

Fig. 2 shows a state where each of ST measurement values of the above-described 12 leads which are acquired for eight hours (8:00 to 16:00) is displayed in a belt-like form.

In Fig. 2, in order to facilitate the understanding, only aVR, II, and aVF of the 12 leads are displayed, and the displays of the other leads are omitted unlike the actual display.

The region indicated by the reference numeral 52 is a simplified event display portion. At a timing when an event displayed on an event item display portion 53 occurs, an event mark (for example, the reference numeral 54) is marked in the simplified event display portion 52.

As the event, ARRHYTHMIA (arrhythmia alarm/event), LIMIT (upper and lower limit alarms), TECHNICAL (technical alarm/event: disconnection of a connector), and OPERATION (operation and intermittent parameter measurement event: blood pressure measurement and the like) are set.

The reference numeral 55 denotes an event cursor. When the operator operates an event skip key 56 to move the event cursor onto a desired event mark, the detail of the event is displayed on a detailed event display portion 57. In Fig. 2, the event cursor 55 is on the event mark 54, and it is shown that the ST value of lead waveform (II) exceeded the upper limit of 0.40 mV at 12:48 of October 24, 2007, and the upper limit alarm was issued.

The displays of aVR, II, and aVF in Fig. 2 are shown in terms of black/white gray scales. When such a display is performed while allocating a plurality of color gradations (for example, 256 gradations) in accordance with ST measurement values, the display can be visually discriminated more easily.

When unique color gradations are set to abnormal values of respective ST measurement values, visibility of an abnormal state can be facilitated.

The display sequence of the 12 leads is not restricted to that of Fig. 2. The displays may be arranged in a simple sequence of I to III, V1 to V6, and the like, or in a sequence considering Cabrera lead.

It is a matter of course that, in addition to the collective display of 12-lead ST measurement values constituting electrocardiogram information of Fig. 2, the displaying unit 5 displays other information (for example, an electrocardiogram, a pulsewave, and a pulse rate) measured by the biological information acquisition apparatus, on the same screen.

Next, the displays of the ST measurement values will be described with reference to the enlarged view of Fig. 3, because, in the display mode of Fig. 2, the displays of the ST measurement values of each lead extend over a long term or eight hours on the same screen, and hence the displays of the ST measurement values are hardly distinguished from each other.

Fig. 3 is a diagram enlargedly showing a part of the ST measurement values of one of the 12 leads (in this case, lead waveform (III)) on the time axis.

The contents of the displays in Fig. 3 do not coincide with those in Fig. 2.

Fig. 3 shows that color gradations are allocated to the ST measurement values, respectively.

Referring to Fig. 3, in zone (a), four ST measurement values are -0.40 mV, and therefore four thin broken lines are displayed, and, in next zone (b), eight ST measurement values are -0.5 mV, and therefore eight thick broken lines are displayed.

In next zone (c), then, five ST measurement values are +0.40 mV, and therefore five thin solid lines are displayed, and, in next zone (d), five ST measurement values are +0.45 mV, and therefore five intermediate solid lines are displayed. In next zone (e), three ST measurement values are +0.5 mV, and therefore three thick solid lines are displayed, and, in next zone (f), eight ST measurement values are +0.40 mV, and therefore eight thin solid lines are displayed.

As described above, for the ST measurement values, displays of lines to which the color gradations are allocated are continuous along the time axis, whereby a display mode showing the trend over a long time period as indicated in Fig. 2 can be acquired.

In principle, a color gradation based on each of ST measurement values is allocated to the each line display of Fig. 3. Alternatively, a color gradation in the form of one line may be allocated to an average of plural ST measurement values.

According to an aspect of the invention, it is possible to realize a method and apparatus for collectively displaying 12-lead ST measurement values in which 12-lead ST measurement values of electrocardiogram information are collectively displayed on a same display screen in a mutually distinguishable manner, so that it is easy to know which lead (portion of the heart) a disease such as ischemic heart disease occurs in.

## Claims

1. A method of displaying a plurality of ST values constituting biological information of a subject displayed on a screen, the method comprising:
defining color gradations corresponding to degrees of the plurality of ST values to be acquired;
acquiring the plurality of ST values from the subject;
determining display elements for the acquired plurality of ST values in accordance with the defined color gradations; and
displaying the determined display elements along a time axis in a belt-like form on the screen, for each of the plurality of ST values.

2. The method according to claim 1, wherein
the biological information is electrocardiogram information, and
the plurality of ST values are 12-lead ST measurement values.

3. The method according to claim 1, wherein
the plurality of ST values include a normal ST value and an abnormal ST value, and
the color gradation of the abnormal ST value is clearly different from the color gradation of the normal ST value.

4. A biological information display apparatus which is configured to display 12-lead ST measurement values constituting electrocardiogram information of a subject on a screen, the biological information display apparatus comprising:
a color gradation setting unit, configured to define color gradations corresponding to degrees of the 12-lead ST measurement values to be acquired;
an ST measurement value acquiring unit, configured to acquire the 12-lead ST measurement values from the subject;
a color gradation allocating unit, configured to determine display elements for the acquired 12-lead ST measurement values in accordance with the defined color gradations; and
a displaying unit, configured to display the determined display elements along a time axis in a belt-like form on the screen, for each of the 12-lead ST measurement values.
